(19) Europäisches Patentamt
European Patent Office
Office européen des brevets

(11) Publication number: **0 156 279 B1**

# (12) EUROPEAN PATENT SPECIFICATION

(45) Date of publication of patent specification: **13.09.89**

(51) Int. Cl.$^4$: **C 07 C 103/30**, C 07 D 263/22

(21) Application number: **85103104.7**

(22) Date of filing: **18.03.85**

## (54) A novel process for the preparation of bestatin derivatives and intermediates thereof.

(30) Priority: **26.03.84 JP 56137/84**

(43) Date of publication of application:
**02.10.85 Bulletin 85/40**

(45) Publication of the grant of the patent:
**13.09.89 Bulletin 89/37**

(84) Designated Contracting States:
**AT BE CH DE FR GB IT LI LU NL SE**

(56) References cited:
**CHEMICAL ABSTRACTS, vol. 85, no. 17, 25 Oct 1978, Columbus, OH (US); p. 643, no. 123901g**
**TETRAHEDRON LETTERS, vol. 25, no. 44, 1984, pp. 5079-5082, Pergamon Press, Oxford, GB; S. KOBAYASHI et al.: "A stereocontrolled synthesis of (-)-bestatin from an acyclic allylamine by iodocyclocarbamation"**

(73) Proprietor: **MICROBIAL CHEMISTRY RESEARCH FOUNDATION**
**14-23, Kamiosaki 3 Chome Shinagawa-ku**
**Tokyo 141 (JP)**

(72) Inventor: **Umezawa, Hamao, Prof.**
**4-23, Toyotamakita Nerimaku**
**Tokyo (JP)**
Inventor: **Ohno, Masaji**
**1565-13, Koshigoe**
**Kamakura City Tokyo (JP)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 156 279 B1

**Description**

This invention relates to a new method of preparing bestatins and intermediates thereof. More particularly, the invention relates to a new method of preparing bestatins of the general formula

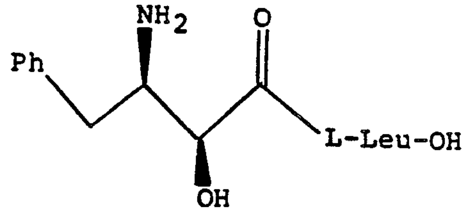

(I)

wherein Ph represents a phenyl group; which may be substituted 1—3 times preferably, one time by hydroxy, $C_1$—$C_3$-alkyl or $C_1$—$C_3$-alkoxy, preferably by hydroxy, methyl or methoxy; and Leu represents a leucine residue, characterized in that a (4R,5S)-4-benzyl-5-alkoxycarbonyloxazolidine-2-one of the general formula

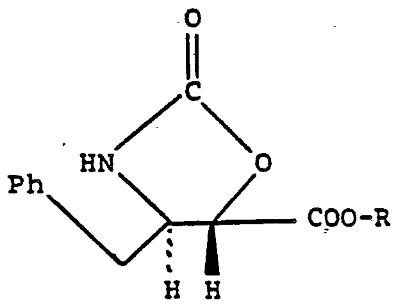

(II)

wherein Ph is as defined above and R represents a lower alkyl group is subjected to the opening of the oxazolidine ring and to the elimination of the lower alkyl group so as to form the corresponding (2S,3R)-3-amino-2-hydroxy-4-phenylbutyric acid, and after protecting the amino group of the so-formed phenylbutyric acid, the amino-protected phenylbutyric acid or its reactive derivative is condensed with carboxyl protected L-leucine or its salt and then the protecting groups are removed from the condensate. The invention relates further to an intermediate, (4R,5S)-4-benzyl-5-alkoxycarbonyloxazolidine-2-one of the general formula

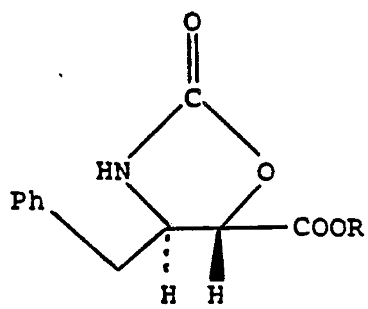

(III)

wherein Ph represents a phenyl group which may be substituted in formula I and R represents a lower alkyl group.

Bestatin and its derivatives are already known from Japanese Patent Publication (Kokoku) Sho 52—33193. They have anti-tumor activity and can, therefore, be used as medicaments.

The method according to the present invention is in the following described in detail:

The opening of the oxazolidine ring and the elimination of the lower alkyl group in the compounds of the general formula (II) are usually carried out by hydrolysis with an acid or alkali. Examples of the alkali include alkali metal hydroxides such as lithium hydroxide, sodium hydroxide, and barium hydroxide, lithium hydroxide being preferred. Examples of the acid include mineral acids such as hydrochlorid acid, and sulfuric acid. The solvent which may be used in the process of the present invention is water or a mixture of water and an inert organic solvent, preferably a lower alcohol such as methanol or ethanol or acetone, tetrahydrofuran, dioxane, dimethylformamide, or dimethylacetamide. The reaction temperature is not critical, but the reaction is usually carried out at a temperature between 0°C and 160°C, preferably between room temperature and 120°C.

Under these conditions, the opening of the oxazolidne ring and the elimination of the lower alkyl group generally take place at the same time, but this is not critical.

The (2S,3R)-3-amino-2-hydroxy-4-phenylbutyric acid obtained from the corresponding oxazolidine-2-

one of the general formula (II) may be isolated, but the protection of the amino group therein can be accomplished without isolation.

The protection of the amino group can be carried out by a conventional method.

Thus, the protection of the amino group with a benzyloxycarbonyl group may be performed by reacting said phenylbutyric acid with benzyloxycarbonyl chloride in the presence of an alkali according to the Schotten-Baumann method or by reacting said phenylbutyric acid with a benzyloxycarbonylating agent such as benzyloxycarbonyl p-nitrophenyl ester, benzyloxycarbonyl azide, benzyloxycarbonyl N-hydroxysuccinimide ester, benzyl S-4,6-dimethylpyrimido-2-yl-thiocarbonate in the presence of an organic tertiary amine such as triethylamine or N-methylmorpholine in a mixture of water and a lower alcohol, dioxane, tetrahydrofuran, acetonitrile or dimethylformamide.

Any condensation method used for the formation of the peptide bond in peptide art is applicable to the condensation of said amino-protected phenylbutyric acid or its reactive derivative with carboxyl-protected L-leucine or its salt.

The term "reactive derivative" means the derivative haging a carboxyl group so activated as to react with the amino group to form an acid imide bond.

Examples of condensation methods include the carbodiimide method using dicyclohexylcarbodiimide or 1-ethyl-3-(3-dimethylaminopropyl)-carbodiimide; the azide method from hydrazide; the mixed acid anhydride method using ethyl chlorocarbonate or isobutyl chlorocarbonate; the active ester method using cyanomethyl ester, vinyl ester, substituted or unsubstituted phenyl ester, thiophenyl ester, or hydroxysuccinimide ester; the O-acrylhydroxylamine derivative method using acetoxime or cyclohexanonoxime; or the N-acyl compound method using carbodiimidazole.

Any solvent used in conventional peptide condensation can be used as a solvent in the condensation of the present invention. Examples of solvents which may be used include ethers such as diethyl ether, tetrahydrofuran and dioxane; esters such as ethyl acetate; ketones such as acetone and methyl ethyl ketone; halogenated hydrocarbons such as methylene chloride and chloroform; amides such as dimethylformamide and dimethylacetamide; or nitriles such as acetonitrile.

The elimination of the protectng group after completing the condensation reaction can be carried out by any conventional method, that is known in the peptide art and which is suited to the specific protecting group present, such as, for example, catalytic reduction using palladium catalyst; treatment with hydrogen bromide in acetic acid; treatment with trifluoroacetic acid; treatment with hydrogen chloride in an organic solvent; saponification with an alkali; treatment with hydrazine; treatment with metallic sodium in liquid ammonia; or treatment with liquid hydrogen fluoride.

The compounds of the general formula (II) used as the starting compound in the present invention include (4R,5S)-4-benzyl-5-methoxycarbonyloxazolidine-2-one, (4R,5S)-4-p-hydroxybenzyl-5-methoxycarbonyloxazolidine-2-one, and (4R,5S)-4-p-methoxybenzyl-5-methoxycarbonyloxazolidine-2-one.

When the phenyl group is substituted with a functional group such as a hydroxy group, the substituent may be protected prior to the condensation reaction with leucine, as required.

The compounds of the general formula (I) obtained according to the present invention include, for example, (2S,3R)-3-amino-2-hydroxy-4-phenylbutanoyl-(S)-leucine (bestatin), (2S,3R)-3-amino-2-hydroxy-4-(p-hydroxyphenyl)butanoyl-(S)-leucine (p-hydroxybestatin), and (2S,3R)-3-amino-2-hydroxy-4-p-methoxyphenylbutanoyl-(S)-leucine (p-methoxybestatin).

According to the process of the present invention, the phenylbutyric acid can be prepared from the corresponding compound of the general formula (II) above without forming any racemate. Thus, the process of the present invention is very suitable for the synthesis of bestatins.

The compound of the general formula (II) used as the starting compound in the present invention may be prepared in the following manner:

A lower alkyl ester of N-benzyloxycarbonyl-(R)-phenylalanine such as, for example, ethyl ester of N-benzyloxycarbonyl-(R)-phenylalanine, ethyl ester of N-benzyloxycarbonyl-(R)-p-hydroxyphenylalanine, ethyl ester of N-benzyloxycarbonyl-(R)-p-methoxyphenylalanine is reduced with a metal halide such as, for example, diisobutyl aluminum hydride (hereunder referred to as "DIBAL—H"), lithium aluminum halide, lithium butoxyaluminum halide, sodium dihydrobismethoxyethoxy aluminate to form the corresponding aldehyde and the so-formed aldehyde is reacted with methylenetriphenylphosphorane obtained from methyltriphenylphosphonium iodide and potassium hydride form the corresponding phenylbutene such as, for example, 3R-benzyloxycarbonyl-amino-4-phenyl (or -p-hydroxyphenyl- or -p-methoxyphenyl)-butene and then the so-obtained phenylbutene is reacted with benzyl bromide to protect the amino group as aminobenzyl and finally the amino protected phenylbutene is reacted with iodine to form the corresponding (4R,5S)-iodomethyl-oxazolidine-2-one.

After reacting the (4R,5S)-iodomethyl-oxazolidine-2-one with silver acetate, the reaction product is treated with a mixture of methanol and 1 N sodium hydroxide or reacted with silver trifluoro-acetate and then the obtained product is treated with 2 N HCl to form the corresponding 5-hydroxymethyloxazolidine-2-one. This product is reacted with metallic sodium in liquid ammonia to eliminate the benzyl group at 3-position. The so-formed compound such as, for example, (4R,5S))-4-benzyl (or -p-hydroxybenzyl or -p-methoxybenzyl)-5-hydroxymethyloxazolidine-2-one is oxidized with an oxidizing agent such as Jones' reagent to form the corresponding carboxylic acid, which is esterified with a conventional esterifying agent such as, for example, diazo lower alkane, sulfuric acid/lower alcohol, gaseous hydrogen chloride/lower

alcohol, thionyl chloride/lower alcohol to form the compound of the general formula (II).

The present invention will be further illustrated by the following examples.

Example 1

(1) (2S,3R)-3-benzyloxycarbonylamino-2-hydroxy-4-phenylbutyric acid

To a solution of 700 mg (3.0 mmol) of (4R,5S)-4-benzyl-5-methoxycarbonyloxazolidine-2-one in 10 ml of methanol was added 10 ml of aqueous 2 N lithium hydroxide and the mixture was heated for 6 hours under reflux in an oil bath (110°C). After adjusting the pH to 7 with 2 N HCl, methanol was distilled off. After readjusting the pH 7, 100 ml of distilled water was added and the amino group was exchanged and entrapped with 70 ml of Amberlite IR—120 B® ($H^+$ type). After washing thoroughly with about one liter of distilled water, elution was conducted with 2 N aqueous ammonia. The eluate was concentrated to about 30 ml and the pH was adjusted to 7. To this was added 10 ml of ether, followed by 3 ml of aqueous 1 N sodium hydroxide, the mixture being cooled in an ice bath. To the ice-cold, vigorously stirred mixture were added 4.45 ml of aqueous 1 N sodium hydroxide solution and 640 μl (4.45 mmol) of benzyloxycarbonyl chloride in three portions within one hour. The stirring was continued for one hour with cooling in an ice bath and at room temperature for another three hours. The reaction mixture was made basic and extracted twice with 30 ml portions of ether. The aqueous phase was acidified to pH = 1 with 2 N HCl and extracted three times with 100 ml portions of ethyl acetate. The combined ethyl acetate layers were washed with saturated aqueous NaCl solution and dried over anhydrous sodium sulfate. The solvent was distilled off and the residue was dried under reduced pressure to give 704 mg (2.12 mmol) of the title compound.

Yield: 72%.

An analytical sample was obtained by recrystallisation from ethyl acetate/hexane.

m.p.: 161.0—161.5°C (lit. 154—155°C).

I.R.: 3300, 1705, 1638 $cm^{-1}$.

(2) [(2S,3R)-3-benzyloxycarbonylamino-2-hydroxy-4-phenylbutanoyl]-L-leucine benzyl ester

Four hundred and six mg (1.24 mmol) of (2S,3R)-3-benzyloxycarbonylamino-2-hydroxy-4-phenylbutyric acid obtained in (1) above, 609 mg (1.48 mmol, 1.2 eq.) of L-leucine benzyl ester toluenesulfonate salt, 200 mg (1.48 mmol, 1.2 eq.) of 1-hydroxybenzotriazole and 206 μl (1.48 mmol, 1.2 eq.) of triethylamine were dissolved into 10 ml of tetrahydrofuran. To this solution was added an ice-cold solution of 281 mg (1.36 mmol, 1.1 eq.) of dicyclohexylcarbodiimide in 10 ml of methylene chloride with stirring and the stirring was continued overnight, after which time the mixture was concentrated. To the residue was added ethyl acetate and the mixture was filtered through a cotton plug. The ethyl acetate layer was extracted successively with 1 N sulfuric acid, water, 2% sodium bicarbonate, and water, dried over $Na_2SO_4$, and then concentrated. The residue was recrystallized from ethyl acetate/hexane to give 630 mg (1.18 mmol) of the title compound.

Yield: 95%.

m.p.: 127.0—127.5°C (lit. 122—123°C).

I.R.: 3400, 1740, 1710, 1655, 1535, 1510 $cm^{-1}$.

(3) [(2S,3R)-3-amino-2-hydroxy-4-phenbutanoyl]-L-leucine (bestatin)

To a mixture of 309 mg (0.58 mmol) of [(2S,3R)-3-benzyloxycarbonylamino-2-hydroxy-4-phenylbutanoyl]-L-leucine benzyl ester and 30 mg of Pd-black was added 15 ml of methanol/water (2/1) and the mixture stirred under a hydrogen atmosphere overnight. The reaction mixture was filtered thorugh Celite® and the filtrate was concentrated to afford 179 mg (0.58 mmol) of the title compound.

Yield: quantitative.

m.p.: 232—233°C (lit. 233—236°C).

I.R.: 3200, 2950, 1690, 1635, 1533 $cm^{-1}$.

M.S.: 308 $[M^+]$, 217 $[M - PhCH_2]^+$.

Referential Example

(1) 3(R)-benzyloxycarbonylamino-4-phenylbutene

To an ice-cold suspension of 63.6 g (0.158 mol) of methyltriphenylphosphonium iodide in 300 ml of tetrahydrofuran (THF) was added 23.5 mg (0.135 mmol) of potassium hydride (23% suspension in mineral oil) under an argon atmosphere and the mixture stirred at room temperature overnight. To this reaction mixture was added 150 ml of dry toluene and the mixture allowed to stand to form a solution of methylentriphenylphosphorane in THF/toluene.

N-Benzyloxycarbonylphenylalanine ethyl ester in an amount of 15.4 g (47 mmol) was dissolved in 200 ml of dry toluene under an argon atmosphere and the resulting solution was chilled to −78°C in a dry ice/ethanol bath. To this chilled solution was added dropwise 100 ml of diisobutylaluminium hydride (1.0 M solution in toluene) with vigorous stirring over 30 minutes. After stirring for another 30 minutes, 200 ml of 2N HCl was added to the reaction mixture. The resulting mixture was allowed to warm to room temperature and extracted three times with 300 ml portions of ethyl acetate. The combined organic layers were washed with water and dried over anhydrous sodium sulfate. After concentrating the dried organic phase at below 40°C, to the residue was added 200 ml of dry toluene under an argon atmosphere and the

solution chilled to −78°C in a dry ice/ethanol bath. To this chilled solution was added 340 ml of the previously prepared solution of $Ph_3P=CH_2$ in THF/toluene with vigorous stirring for one hour, and the resultant mixture was stirred at −78°C for one hour, followed by stirring at room temperature for 30 minutes. Thereafter, 200 ml of a saturated aqueous ammonium chloride solution was added. The resulting mixture was extracted three times with 300 ml portions of ethyl acetate. The combined extracts were washed with saturated aqueous sodium chloride solution, dried over anhydrous sodium sulfate and concentrated. The residue was chromatographed over silica gel using first methylene chloride/n-hexane (1/1), then switching to a 2:1 mixture. Recrystallization of the eluates from benzene/hexane gave 8.44 g (3.00 mmol) of the title compound as a white needle.

Yield: 64%.
m.p.: 92.6—93.0°C.
I.R.: (KBr): 3330, 1680, 1525, 1250 $cm^{-1}$.

(2) 3(R)-(N-benzyl-N-benzyloxycarbonyl)amino-4-phenylbutene

Potassium hydride (23% suspension in mineral oil) in an amount of 5.7 ml (32.8 mmol) was repeatedly washed with dry hexane under an argon atmosphere so as to remove the mineral oil. To this was added 20 ml of dry benzene and stirred in an ice bath. To this mixture was added dropwise 7.71 g (27.4 mmol) of 3(R)-benzyloxycarbonyl-amino-4-phenylbutene in 20 ml of dimethylformamide and the mixture was stirred at room temperature for 30 minutes, after which 3.91 ml (32.9 mmol) of benzyl bromide was added with cooling in an ice bath and the mixture stirred at room temperature for 30 minutes. The reaction mixture was poured into cold water and extracted three times with 100 ml portions of ethyl acetate. The combined organic layers were washed with saturated aqueous sodium chloride solution dried over anhydrous sodium sulfate and concentrated. The residue was chromatographed over silica gel using ether/hexane (1:6) as an eluent to give 9.7 g (26.0 mmol) of the title compound as a colorless oil.

Yield: 95%.
IR.: 1695 $cm^{-1}$.
M.S.: 372 $(M + 1]^+$, 281 $[M - PhCH_2 + 1]^+$, 236 $[M - Z]^+$.
$[\alpha]_D^{20} = -80.4$°C (C = 2.7, $CHCl_3$).
Rf (TLC on silica gel) = 0.44 (ether/hexane = 1:4).

(3) (4R,5S)-3,4-dibenzyl-5-iodomethyloxazolidine-2-one

To an ice-cold solution of 160 mg (0.043 mmol) of 3(R)-(N-benzyl-N-benzyloxycarbonyl)amino-4-phenylbutene in 2 ml of methylene chloride was added 330 mg (1.3 mmol) of iodine with stirring. The mixture was stirred in an ice bath for 4 hours, after wich an aqueous sodium thiosulfate solution was added and the resulting mixture was extracted twice with 30 ml portions of methylene chloride. The combined layers were washed with saturated aqueous sodium chloride solution, dried over anhydrous sodium sulfate and concentrated. The residue was chromatographed over silica gel, eluting with ether/hexane (1:1) to give 141 mg (0.347 mmol) of the title compound and 21 mg (0.051 mmol) of the (5R)-isomer.

Yield: 92% (5S:5R = 7:1).

An analytical sample was obtained by recrystallization from ether/hexane.

(4R,5S)-compound:
m.p.: 110.2—110.8°C.
I.R.: 1750, 1490, 1450, 1420 $cm^{-1}$.
$[\alpha]_D^{20} = +34.2$°C (C = 2.4, $CHCl_3$).
Rf (TLC on silica gel) = 0.27 (ether/hexane = 1:1).

(4) (4R,5S)-3,4-dibenzyl-5-hydroxymethyloxazolidine-2-one

*Method 1*

To a solution of 7.4 g (18.2 mmol) of (4R,5S)-3,4-dibenzyl-5-iodomethyloxazolidine-2-one in 30 ml of dimethylformamide and 50 ml of acetic acid was added (6.1 g) (36.3 mmol) of silver acetate under an argon atmosphere and the mixture stirred at 130°C for 5 hours. The solvent was removed under reduced pressure and the residue was filtered through a cotton plug. The filtrate was subjected to azeotropic distillation with toluene to remove acetic acid and dimethylformamide. To the residue were added 100 ml of emthanol and 30 ml of 1 N aqueous sodium hydroxide solution and the mixture stirred at room temperature for one hour. After adjusting the pH to 7 with aqueous 2 N HCl solution, methanol was distilled off under reduced pressure. The residue was extracted three times with 100 ml portions of ethyl acetate. The combined extracts were washed with saturated aqueous sodium chloride solution, dried over anhydrous sodium sulfate and concentrated. The residue was chromatographed over 40 g of silica gel with ether/hexane (4:1) elution. Recrystallization from benzene/hexane gave 5.01 g of the title compound.

Yield: 93%.

*Method 2*

To a solution of 21.5 mg (0.053 mmol) of (4R,5S)-3,4-dibenzyl-5-iodomethyloxazolidine-2-one in 0.5 ml of DMF was added 35.0 mg (0.16 mmol) of silver trifluoroacetate under an argon atmosphere and the

mixture stirred at room temperature for 3 days, after which period water was added to the reaction mixture and the mixture extracted three times with 20 ml portions of ethyl acetate. The combined organic layers were washed with saturated aqueous sodium chloride solution, dried over anhydrous sodium sulfate and concentrated. To the residue was added 2 ml of 2 N HCl and stirred at room temperature for 5 hours. After adjusting the pH to 7 with 1 N NaOH, the mixture was extracted three times with 10 ml portions of ethyl acetate. The combined extracts were washed with saturated aqueous NaCl solution, dried over anhydrous sodium sulfate and concentrate. The resulting residue was purified by preparation thin layer chromatograph developing with ether to give 13.0 mg (0.044 mmol) of the title compound.

Yield 83%.
m.p.: 96.9—97.5°c.
I.R.: 3390, 1700, 1445, 1435 $cm^{-1}$.
M.S.: 389 $[M + PhCH_2]^+$, 298 $[M + 1]^+$, 266 $(M - CH_2OH]^+$, 206 $[M - PhCH_2]^+$.
$[\alpha]_D^{20} = +45.5°C$ (C = 1.1, $CHCl_3$).
Rf (TLC on silica gel) = 0.39 (ether).
Analysis:

| | | | |
|---|---|---|---|
| Calc'd. for $C_{18}H_{19}NO_3$: | N 4.71%; | C 72.71%; | H 6.44% |
| Found: | N 4.47%; | C 72.63%; | H 6.46%. |

(5) (4R,5S)-4-benzyl-5-hydroxymethyl-oxazolidine-2-one

(4R,5S)-3,4-dibenzyl-5-hydroxymethyloxazolidine-2-one in an amount of 5.0 g (17.0 mmol) was chilled to −78°C in a dry ice/ethanol bath under an argon atmosphere. To this was added about 100 ml of dry ammonia through a soda lime tube. To the resulting mixture was added metallic sodium with vigorous stirring until blue color was fixed. The reaction mixture was stirred for 1.5 hours with refluxing ammonia using an ethanol bath in place of the dry ice/ethanol bath. After chilling to −78°C, crystalline ammonium chloride was added to the chilled mixture in small portions to remove excess sodium. The reaction mixture was allowed to stand at room temperature. After removing ammonia, 100 ml of water was added to the reaction mixture and the mixture extracted three times with 100 ml portions of ethyl acetate. The combined organic layers were washed with saturated aqueous NaCl solution, dried over anhydrous sodium sulfate, and concentrated. The residue was purified by column chromatography eluting with first ethyl acetate/hexane (2:1) and then with a (4:1) mixture. The eluates were recrystallized from benzene/hexane to give 2.90 g (14.0 mmol) of the title compound.

Yield: 82%.
m.p.: 88.5—89.3°C.
I.R.: 3440, 3280, 2748, 1700, 1415, 1250 $cm^{-1}$.
N.M.R.: ($CDCl_3$ containing a small amount of $D_2O$)
2.8 (2H, m, $PhCH_2$)
3.4 (1H, m, 5 − H)
3.8 (2H, m, $—CH_2OH$)
4.3 (1H, m, 4 − H)
5.5 (1H, b, —NH)
7.2 (5H, m, aromatic).
M.S.: 208 $[M + 1]^+$, 116 $[M - PhCH_2]^+$.
$[\alpha]_D^{23} = -89.8°C$ (C = 0.83, $CHCl_3$).
Rf (TLC on silica gel) = 0.31 (ether acetate/hexane = 4:1).
Analysis:

| | | | |
|---|---|---|---|
| Calc'd. for $C_{11}H_{13}NO_3$: | N 6.76%; | C 63.76%; | H 6.32% |
| Found: | N 6.55%; | C 63.56%; | H 7.18%. |

(6) (4R,5S)-4-benzyl-5-methoxycarbonyl-oxazolidine-2-one

(4R,5S)-4-Benzyl-5-hydroxymethyloxazolidine-2-one in an amount of 224.7 mg (1.1 mmol) was dissolved in 15 ml of acetone distilled from potassium permanganate. To this was added 2 ml of Jones' reagent equivalent to 8 normals as an active oxygen, followed by vigorous stirring that lasted overnight. Excess Jones' reagent was decomposed by adding isopropyl alcohol. After distilling off about half the volume of acetone, water was added and the mixture extracted four times with 50 ml portion of ethyl acetate. The combined organic layers were washed with a saturated aqueous NaCl solution, dried over anhydrous sodium sulfate and concentrated. To the residue was added about 2 ml of methanol. To the resulting mixture was added a solution of diazomethane in ether with stirring in an ice bath until the evolution of nitrogen stopped. After distilling off the solvents, the residue was crystallized from diisopropyl ether/hexane to give 138 mg (0.587 mmol) of the title compound.

Yield: 54%.
m.p.: 91.0—91.6°C.
I.R.: 3400, 1750 $cm^{-1}$.
N.M.R.:
2.9 (1H, dd, $J\phi\phi = 13.5$, $J\phi_4 = 7.9$, $H\phi$)
3.0 (1H, dd, $J\phi'\phi = 13.5$, $J\phi'_4 = 5.8$, $H\phi'$)

3.79 (3H, s, $—CO_2Me$)
4.1 (1H, m, $H_4$).
In case or irradiation with amine proton,
(1H, dd, $J_{4\Phi} = 7.9$, $J_{4\Phi'} = 5.8$, $J_{45} - 4.6$)
4.7 (1H, d, $J_{54} = 4.6$, $H_5$)
5.9 (1H, b, —N*H*)
7.2 (5H, m, aromatic).
M.S.: 235 $[M]^+$, 203 $[M - MeOH]^+$, 144 $(M - PhCH_2]^+$.
$[\alpha]_D^{23} = +66.3°C$ (C = 1.2, $CHCl_3$).
Rf (TLC on silica gel) = 0.43 (ether acetate/hexane = 2:1).
Analysis:

| | | | |
|---|---|---|---|
| Calc'd. for $C_{12}H_{13}NO_4$: | N 5.95%; | C 61.27%; | H 5.57% |
| Found: | N 5.90%; | C 61.49%; | H 5.59%. |

## Claims

1. A method of preparing bestatins of the general formula

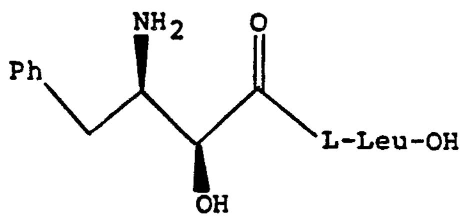

(I)

wherein Ph represents an optionally substituted phenyl group; and Leu represents a leucine residue, characterized in that a (4R,5S),-4-benzyl-5-alkoxycarbonyloxazolidine-2-one of the general formula

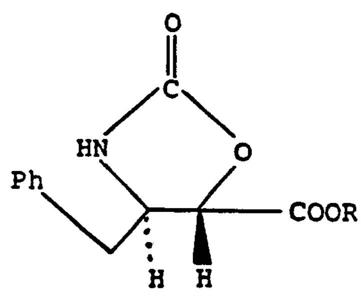

(II)

wherein Ph is as defined above and R represents a lower alkyl group is subjected to the opening of the oxazolidine ring and to the elimination of the lower alkyl group so as to form the corresponding (2S,3R)-3-amino-2-hydroxy-4-phenylbutyric acid, and after protecting the amino group of the so-formed phenylbutyric acid, the phenylbutyric acid or its reactive derivative is condensed with a carboxyl-protected L-leucine or its salt and then the protecting groups are removed from the condensate.

(2) A (4R,5S)-4-benzyl-5-alkoxycarbonyloxazolidine-2-one of the general formula

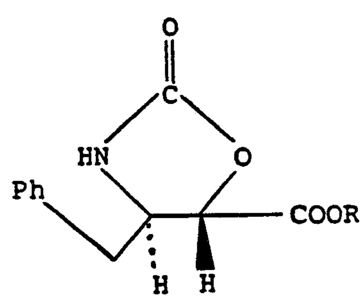

(II)

wherein Ph represents an optionally substituted phenyl group and R represents a lower alkyl group.

## Patentansprüche

1. Verfahren zur Herstellung von Bestatinen der allgemeinen Formel

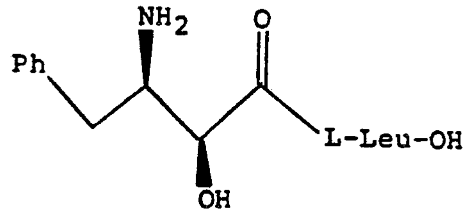

(I)

worin Ph eine gegebenenfalls substituierte Phenylgruppe und Leu einen Leucinrest bedeutet, dadurch gekennzeichnet, dass ein (4R,5S)-4-Benzyl-5-alkoxycarbonyloxazolidin-2-on der allgemeinen Formel

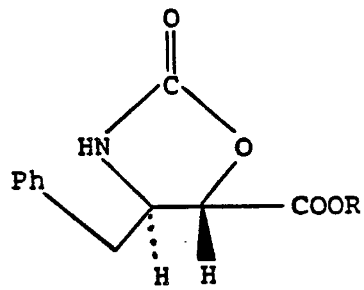

(II)

worin Ph wie vorher definiert ist und R eine niedere Alkylgruppe bedeutet, der Öffnung des Oxazolidinringes und der Eliminierung der niederen Alkylgruppe unterworfen wird, um die entsprechende (2S,3R)-5-Amino-2-hydroxy-4-phenylbuttersäure zu bilden und nach dem Schützen der Aminogruppe der so gebildeten Phenylbuttersäure, die Phenylbuttersäure oder ihr reaktives Derivat mit einem carboxylgeschützten L-Leucin oder seinem Salz kondensiert wird und hierauf die Schutzgruppen von dem Kondensat entfernt werden.

2. Ein (4R,5S)-4-Benzyl-5-alkoxycarbonyloxazolidin-2-on der allgemeinen Formel

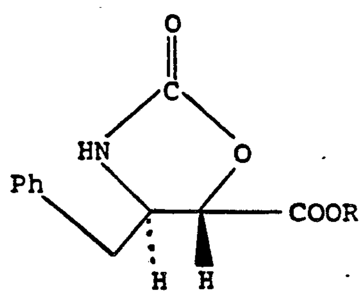

(II)

worin Ph eine gegebenenfalls substituierte Phenylgruppe und R eine niedere Alkylgruppe bedeutet.

**Revendications**

1. Procédé pour préparer des bestatines de formule générale

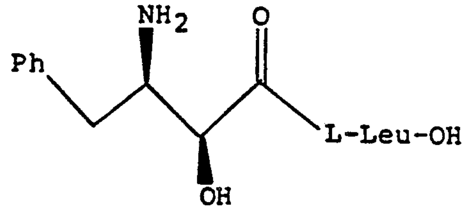

(I)

dans laquelle Ph représente un groupe phényle éventuellement substitué; et Leu représente un reste de leucine, caractérisé en ce que l'on soumet une (4R,5S)-4-benzyl-5-alcoxycarbonyloxazolidine-2-one de formule générale

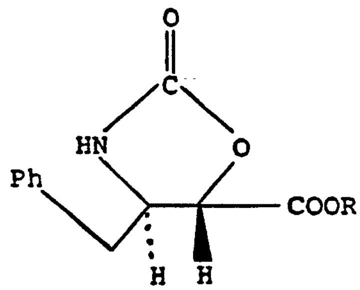

(II)

dans laquelle Ph est comme défini ci-dessus et R représente un groupe alkyle inférieur, à l'ouverture du cycle oxazolidine et à l'élimination du groupe alkyle inférieur, de façon à former l'acide (2R,3R)-3-amino-2-hydroxy-4-phénylbutyrique correspondant et, aprés protection du groupe amino de l'acide phénylbutyrique ainsi formé, on condense l'acide phénylbutyrique ou son dérivé réactive avec une L-leucine carboxy-protégée ou un de ses sels, puis on élimine les groupes protecteurs du condensat.

2. (4R,5S)-4-benzyl-5-alcoxycarbonyloxazolidine-2-one de formule générale

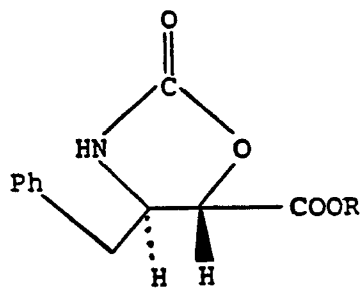

(II)

dans laquelle Ph représente un groupe phényle éventuellement substitué et R représente un groupe akyle inférieur.